(19) Europäisches Patentamt
European Patent Office
Office européen des brevets

(11) EP 2 544 009 A2

(12) EUROPEAN PATENT APPLICATION
published in accordance with Art. 153(4) EPC

(43) Date of publication:
**09.01.2013 Bulletin 2013/02**

(21) Application number: **10836158.5**

(22) Date of filing: **26.11.2010**

(51) Int Cl.:
***G01N 35/00*** *(2006.01)* ***G01N 21/47*** *(2006.01)*
***G01N 33/53*** *(2006.01)*

(86) International application number:
**PCT/KR2010/008448**

(87) International publication number:
**WO 2011/071262 (16.06.2011 Gazette 2011/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.12.2009 KR 20090123007**

(71) Applicant: **Nanoentek Inc.**
**Seoul 152-711 (KR)**

(72) Inventors:
- **HUR, Dae Sung**
  **Suwon-si**
  **Gyeonggi-do 440-320 (KR)**
- **OH, Jong Hyun**
  **Seoul 134-793 (KR)**
- **KIM, Jae Jeong**
  **Seoul 150-828 (KR)**
- **PARK, Eun Hee**
  **Seoul 151-854 (KR)**

(74) Representative: **Breesé, Pierre**
**Fidal Innovation**
**32, place Ronde**
**92035 Paris la Defense cedex (FR)**

# (54) MICROFLUIDIC DEVICE COMPRISING MICROCHANNEL WHERE PROTRUSIONS ARE FORMED ON BOTTOM SURFACE

(57) Disclosed is a microfluidic device comprising a microchannel through which fluid can flow. Protrusions are formed on the bottom surface of the microchannel. The microfluidic device increases detection sensitivity by improving optical characteristics and enhances the reactivity of biochemical reaction by slowing down the velocity of fluid flowing inside the microchannel.

[FIG. 2a]

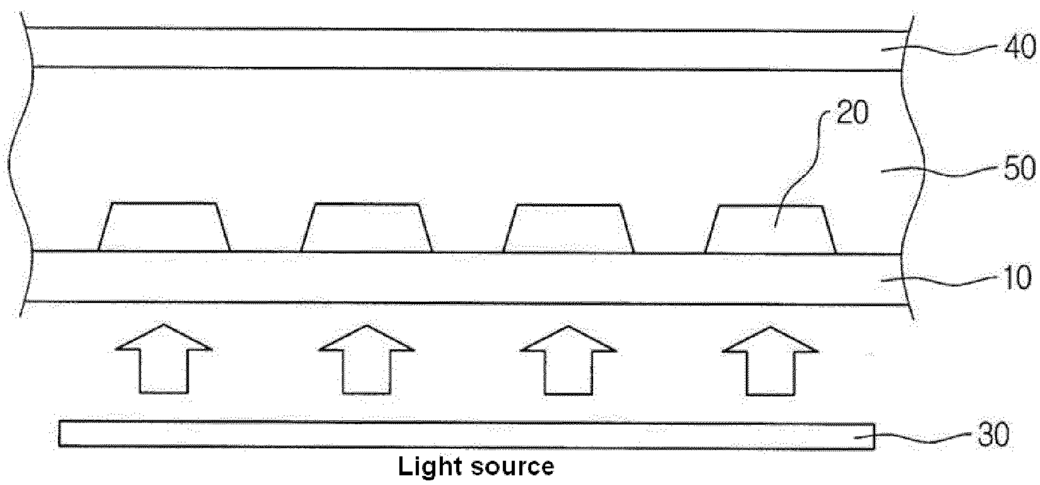


EP 2 544 009 A2

## Description

### Technical Field

The present disclosure relates to a microfluidic device having a microchannel through which a fluid can flow.

### Background Art

At present, researches and developments are actively made about point-of-care (POC) and lab-on-a-chip (LOC) devices in the field of bioindustry. The LOC is a device that integrates laboratory functions for conducting various experiments for biological, medical and pharmacological studies as a single plastic microfluidic device.

By injecting a sample such as blood into a channel of the microfluidic device or making it flow through the channel, a biochemical reaction is made to occur and the reaction product is analyzed optically or spectroscopically. Therefore, diagnosis of a disease or monitoring of progression of the disease can be achieved conveniently.

FIGS. 1a and 1b are a perspective view and a cross-sectional view of an existing microfluidic device having a microchannel. The microfluidic device comprises an upper substrate 160 and a lower substrate 180, and has a microchannel 100, a flow inlet 120 and a flow outlet 140 therein. A fluid flown in through the flow inlet 120 flows through the microchannel 100.

## Disclosure

### Technical Problem

The present disclosure is directed to providing a microfluidic device having a microchannel wherein a protrusion is formed on a bottom surface.

### Technical Solution

The present disclosure relates to a microfluidic device having a microchannel through which a fluid can flow wherein a protrusion is formed on a bottom surface of the microchannel.

The protrusion may have a hemispherical, conical, truncated conical, pyramidal (a cone having a polygonal base), truncated pyramidal, cylindrical, prismatic (a cylinder having a polygonal base) or polyhedral (e.g., regular polyhedral such as cubic) shape. Specifically, the protrusion may have a truncated conical or hemispherical shape. And, a cross section of the protrusion may be circular, polygonal or clover-shaped, or an indented closed plane.

In an exemplary embodiment of the present disclosure, an aspect ratio of the protrusion may be from 2:1 to 4:1. And, the protrusion may be at least 5 $\mu$m in height.

Advantageously, a tilt angle of a side wall of the protrusion is 50-80°. The tilt angle of the side wall of the protrusion may be larger than a critical angle at which total internal reflection occurs at an interface between the protrusion and an aqueous solution, an organic solution or air.

Advantageously, a tilt angle of a side wall of the protrusion is larger than a critical angle at which total internal reflection occurs at an interface between the protrusion and an aqueous solution, an organic solution or air.

Advantageously, a height of the protrusion is 2-190 $\mu$m. And a height of the microchannel may be 5-200 $\mu$m.

The present disclosure also relates to a method for analyzing a sample, comprising: injecting a sample into the microchannel of the microfluidic device mentioned above; irradiating light from under the microchannel; and analyzing the sample by detecting light emitted from the sample.

### Advantageous Effects

According to the present disclosure, detection sensitivity may be improved in biochemical reactions such as antigen-antibody reaction for analysis of proteins or DNA or enzymatic reaction by enhancing the optical property of the reaction product. Further, the reactivity of the biochemical reactions may be improved by lowering the flow rate at the site of reaction. That is to say, the flow rate may be controlled with the protrusion formed according to the present disclosure.

### Brief Description of the Drawings

FIGS. 1a and 1b are a perspective view and a cross-sectional view of an existing microfluidic device having a microchannel, respectively;

FIG. 2a shows a cross section of a microchannel wherein a protrusion is formed according to the present disclosure and irradiation of light from under a bottom surface of the microchannel;

FIG. 2b shows a photographic image obtained after irradiating light from under the bottom surface of the microchannel as in FIG. 2a;

FIGS. 2c and 2d respectively show a photographic image obtained after irradiating light from under a bottom surface wherein a protrusion with a height of 2 μm and 8 μm is formed;

FIG. 2e compares light intensity after irradiating light from under a bottom surface wherein a protrusion with a height of 2 μm or 8 μm is formed;

FIG. 3a illustrates irradiation of light from above a bottom surface of a microchannel wherein a protrusion is formed according to the present disclosure;

FIG. 3b shows a photographic image obtained after irradiating light from above the bottom surface of the microchannel as in FIG. 3a;

FIGS. 3c and 3d respectively show a photographic image obtained after irradiating light from above a bottom surface wherein a protrusion with a height of 2 μm and 8 μm is formed;

FIG. 3e compares light intensity after irradiating light from under or from above a bottom surface wherein a protrusion is formed;

FIGS. 3f-3i compare fluorescence intensity after irradiating light from under or from above a bottom surface of a microfluidic device wherein a protrusion with a height of 8 μm is formed at different sample concentrations;

FIG. 4a shows a cross-sectional view of a conical protrusion;

FIG. 4b is a photographic image of a bottom surface wherein a conical protrusion is formed;

FIG. 5a shows a cross-sectional view of a hemispherical protrusion;

FIG. 5b is a photographic image of a bottom surface wherein a hemispherical protrusion is formed;

FIG. 6 is a figure for illustrating a critical angle at the interface of different media;

FIG. 7 shows incidence of light from a bottom surface of a protrusion with a wall having a tilt angle of $\alpha$;

FIGS. 8a and 8b show light paths for protrusions having different tilt angles;

FIGS. 9a-9e show light-harvesting effect at varying tilt angles;

FIG. 10 shows photographic images showing experimental result for protrusions having different cross sections; and

FIG. 11 shows an analysis device for irradiating light from under a microfluidic device according to the present disclosure in order to analyze a sample using the microfluidic device.

**Mode for the Invention**

Hereinafter, exemplary embodiments of the present disclosure will be described in detail with reference to the accompanying drawings. However, the present disclosure is not limited by the following embodiments.

FIG. 2a schematically shows a cross section of a microchannel 50 wherein a protrusion is formed according to the present disclosure and irradiation of light from under a bottom surface of the microchannel 50.

The microchannel 50 is formed in a space between an upper substrate 40 and a lower substrate 10. A protrusion 20 is formed on a bottom surface of the microchannel 50, i.e. on the lower substrate 10. In this exemplary embodiment, the protrusion 20 has a truncated conical shape.

Hereinafter, a method of analyzing a biochemical sample using a microfluidic device having the microchannel 50 will be described. First, for example, a primary capture antibody is uniformly coated on the bottom surface of the microchannel 50 and a sample such as blood is made to flow through the microchannel. Then, an antigen included in the sample binds with the capture antibody. The antigen captured by the antibody is reacted again with a fluorescence-labeled secondary antibody to bind the antigen with the secondary antibody. The protrusion 20 serves to increase the surface area of the antigen-antibody reaction.

Then, when light is irradiated from a light source 30 from under the bottom surface of the microchannel, the substance resulting from the binding of the primary capture antibody, the antigen and the secondary antibody exhibits fluorescence due to the fluorescent substance labeled at the secondary antibody. Accordingly, by detecting the fluorescence emitted from the secondary antibody, the antigen included in the sample may be analyzed quantitatively and qualitatively.

In an exemplary embodiment, a solution of 0.001% antibody labeled with a fluorescent nanoparticle may be used as the fluorescence-labeled secondary antibody. FIG. 2b shows a photographic image obtained after irradiating light from under the bottom surface of the microchannel. As seen from FIG. 2b, the fluorescent substance located at the side wall of the conical protrusion 20 exhibits fluorescence of very high intensity. It is because the tilted side wall of the protrusion 20 provides a light-harvesting effect. Accordingly, it is easier to optically detect the fluorescent substance. The light-harvesting effect will be described in detail later.

FIGS. 2c and 2d respectively show a photographic image obtained after irradiating light from under a bottom surface wherein a protrusion with a height of 2 μm and 8 μm is formed. As seen from FIGS. 2c and 2d, the fluorescence intensity is higher when the protrusion when the height of the protrusion is 8 μm (FIG. 2d). The experimental result reveals that improved light intensity is achieved when the height of the protrusion is from 2 μm to 20 μm.

FIG. 2e compares fluorescence intensity (dimensionless) after irradiating light from under a bottom surface wherein a protrusion with a height of 2 μm or 8 μm is formed. The abscissa is a relative distance from an arbitrary reference point.

FIG. 3a illustrates irradiation of light from above the bottom surface of the microchannel 50 wherein the protrusion is formed according to the present disclosure. In this case, it is difficult to optically detect the fluorescent substance since the intensity of the fluorescent light emitted from the fluorescent substance is very weak, as seen from FIG. 3b.

FIGS. 3c and 3d respectively show a photographic image obtained after irradiating light from above a bottom surface wherein a protrusion with a height of 2 μm and 8 μm is formed. As seen from FIGS. 3c and 3d, it is difficult to optically detect the fluorescent substance since the intensity of the fluorescent light emitted from the fluorescent substance is very weak, in both cases where the height of the protrusion is 2 μm and 8 μm. It is because the light-harvesting effect by the protrusion is not achieved when the light is irradiated light from above the bottom surface of the microchannel wherein the protrusion is formed.

FIG. 3e compares fluorescence intensity after irradiating light from under or from above a bottom surface wherein a protrusion with a height of 8 μm is formed. As seen from FIG. 3e, a strong light intensity is observed when the light is irradiated light from under the bottom surface of the microchannel wherein the protrusion is formed.

FIGS. 3f-3i compare fluorescence intensity after irradiating light from under or from above a bottom surface of a microfluidic device wherein a protrusion with a height of 8 μm is formed at different sample concentrations.

FIGS. 3f-3i show a result of performing experiments using the Frend microfluidic device (NanoEnTek, Korea) by the sandwich method, using TnI as an analyte. After injecting a sample to the microfluidic device holding a primary capture antibody, the antigen-antibody reaction was measured according to the sandwich method by measuring the intensity of fluorescence signal using an optical instrument.

In FIGS. 3f-3h, the abscissa is a relative distance from an arbitrary reference point and the ordinate is fluorescence intensity (dimensionless).

The samples contained the analyte (TnI) at concentrations of 24 ng/mL, 12 ng/mL and 6 ng/mL for FIG. 3f, FIG. 3g and FIG. 3h, respectively.

As seen from FIGS. 3f-3h, the light intensity is higher when the light is irradiated from under the bottom surface of the microchannel wherein the protrusion is formed than when the light is irradiated from above the bottom surface. In FIG. 3f, the light intensity is improved by about 21% when the light is irradiated from under the bottom surface as compared to when the light is irradiated from above. In FIG. 3g, the light intensity is improved by approximately 22%, and in FIG. 3h, by about 20%. Accordingly, the light intensity is improved by about 21% on average when the light is irradiated from under the bottom surface than when the light is irradiated from above.

Meanwhile, it can be seen that the mean fluorescence intensity is highest when the concentration of the analyte (TnI) is 24 ng/mL (FIG. 3i).

FIG. 4a shows a cross-sectional view of a truncated conical protrusion formed on a bottom surface of a microchannel according to an exemplary embodiment of the present disclosure. In an exemplary embodiment of the present disclosure, the diameter of the lower base of the truncated cone may be 4-80 μm. And, the aspect ratio (i.e., a:c) of the truncated cone may be about 2:1 to 4:1. In addition, the tilt angle $\alpha$ of the side wall of the truncated cone may be about 50-80°. The diameter b of the upper base may be determined to satisfy the above requirements. The upper base of the truncated cone may be either planar or convex.

The height c of the protrusion may be 2-200 μm, specifically 2-190 μm, more specifically 5-20 μm. And, the height of the microchannel 50 (FIG. 2a) may be at least 5 μm, specifically 10-200 μm.

The protrusion may be formed on a lower substrate 10 (FIG. 2a) which becomes the bottom surface of the microchannel by a microelectromechanical system (MEMS) process based on the semiconductor process, a microfabrication technique or a molding technique.

FIG. 4b is a scanning electron microscopic (SEM) image of a bottom surface of a microchannel wherein truncated conical protrusions as shown in FIG. 4a are formed in lattice form. The diameter of the lower base of the truncated cone is 25 μm and its height is 8 μm.

FIG. 5a shows a cross-sectional view of a hemispherical protrusion formed on a bottom surface of a microchannel according to another exemplary embodiment of the present disclosure. In an exemplary embodiment of the present disclosure, the diameter of the lower base of the hemisphere may be 10-50 μm. And, the aspect ratio (i.e., a:c) of the hemisphere may be about 2:1 to 4:1. The tilt angle $\alpha$ of the side wall of the hemisphere may be about 50°-80°.

FIG. 5b is an SEM image of a bottom surface wherein hemispherical protrusions as shown in FIG. 5a are formed in lattice form. The diameter of the lower base of the hemisphere is 25 μm and its height is 8 μm.

Hereinafter, the light-harvesting effect provided by a protrusion formed on a bottom surface of a microchannel according to the present disclosure will be described.

Light is refracted at an interface of different media. When a ray of light is incident from a medium of a larger refractive index (medium 1) to a medium of a smaller refractive index (medium 2), total internal reflection occurs from a particular angle of incidence. This angle of incidence is called critical angle and is given by Equation 1:

[Equation 1]

$$\theta_c = \arcsin (n_2 / n_1)$$

wherein

$\theta_c$ is the critical angle,
$n_1$ is the refractive index of the medium 1, and
$n_2$ is the refractive index of the medium 2.

As shown in FIG. 6, when the angle of incidence of light incident from the medium with the refractive index n1 to the medium with the refractive index n2 is larger than θc, the light is totally reflected internally. For example, if the medium 1 is poly(methyl methacrylate) (PMMA) having a refractive index of 1.49 and the medium 2 is water having a refractive index of 1.33, the critical angle θc is about 67°. Thus, when light is incident from the medium 1 (PMMA) to the medium 2 (water) with an angle of incidence θ larger than 67°, the light is totally reflected internally.
Table 1 shows refractive indices of various media and their critical angles for water (refractive index = 1.33).
[Table 1]

| Refractive indices of various media and their critical angles for water | | |
|---|---|---|
| Material | Refractive index | Critical angle (°) |
| Poly(methyl methacrylate) (PMMA) | 1.49 | 63 |
| Polystyrene (PS) | 1.59 | 57 |
| Polyamide (PA) | 1.55 | 59 |
| Ethylene propylene (EP) rubber | 1.53 | 60 |
| Polyetherimide (PEI) | 1.558 | 59 |
| Polyethersulfone (PES) | 1.65 | 54 |
| Polycarbonate (PC) | 1.5695 | 58 |
| Topas™ | 1.53 | 60 |
| Glass | 1.45 | 67 |

FIG. 7 shows incidence of light from a bottom surface of a protrusion with a wall having a tilt angle of α. When the tilt angle α is larger than the critical angle $\theta_c$, the light incident from under the bottom surface of the protrusion is totally reflected internally at the side wall and travels to the opposing side wall of the protrusion. As a result, it is easier to excite the fluorescent particle fixed on the side wall of the protrusion.
FIGS. 8a and 8b show paths of light emitted from a fluorescent particle 1 for protrusions having different tilt angles α. Referring to FIG. 8a, the light emitted from the fluorescent particle 1 is incident on the side wall of the protrusion with an angle of incidence θ larger than the critical angle $\theta_c$ and is totally reflected internally toward the bottom surface of the protrusion. In particular, when the tilt angle α of the protrusion is larger than the critical angle, the light incident on the protrusion is totally reflected internally and the light-harvesting effect of light is increased. On the contrary, if the tilt angle α is too small as in FIG. 8b, it is difficult to harvest the light since it is scattered.
FIGS. 9a-9e show light-harvesting effect at varying tilt angles. As seen from the figures, when the tilt angle is 50° or larger, specifically 50-80°, the light emitted from the fluorescent particle 1 is totally reflected internally toward the bottom surface of the protrusion.
Usually, biochemical samples (e.g., blood, body fluid, urine, etc.) or other samples used in experiments tend to be dispersions or solutions with water as dispersion medium or solvent. Thus, refractive indices of various media and their critical angles for water are described in Table 1. However, the present disclosure is also applicable, for example, to organic solutions having, e.g., alcohol as solvent. Therefore, according to the present disclosure, the critical angle may be calculated considering the refractive index of the particular biochemical sample, aqueous dispersion, aqueous solution, organic solution, etc. to be tested to design the protrusion of the microchannel. Also, after removing the sample or solution from the microchannel, experiment may be performed with the reaction product (e.g., antigen fixed in the channel as a result of antigen-antibody reaction). In this case, the critical angle may be calculated considering the refractive index of the protrusion and that of air to design the protrusion of the microchannel.

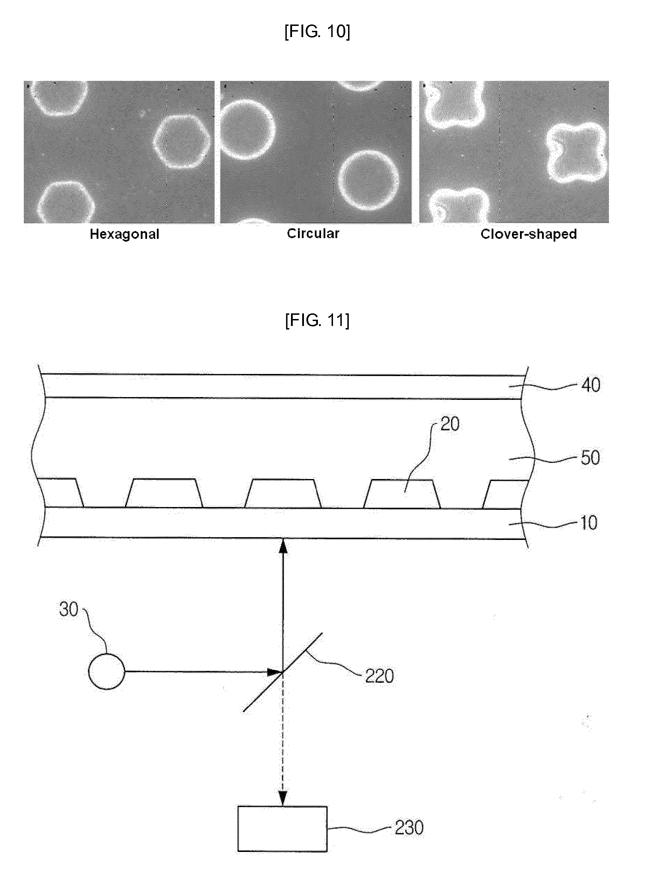

FIG. 10 shows photographic images showing experimental result for protrusions having different cross sections. As seen from the figure, the fluorescence intensity is strongest when the cross section of the protrusion is clover-shaped, followed by circular. The fluorescence intensity is weakest for the hexagonal cross section. It is because the light-harvesting area increases as the circumference of the cross section of the protrusion is larger. In particular, when the cross section of the protrusion has a concave portion such as the clover shape, the light-harvesting effect is improved further since reflected light is superimposed there.

FIG. 11 shows an exemplary analysis device for irradiating light from under a microfluidic device according to the present disclosure in order to analyze a sample using the microfluidic device. In the analysis device, light irradiated from a light source 30 is reflected by a dichroic mirror 220 and is irradiated to a bottom surface of the microfluidic device. And, a fluorescence signal emitted from a sample in the microfluidic device passes through the dichroic mirror 220 and is decoded by an image processor 230.

While the present disclosure has been described with respect to the specific embodiments, it will be apparent to those skilled in the art that various changes and modifications may be made without departing from the spirit and scope of the disclosure as defined in the following claims.

## Claims

1. A microfluidic device having a microchannel through which a fluid can flow, wherein a protrusion is formed on a bottom surface of the microchannel.

2. The microfluidic device according to claim 1, wherein the protrusion has a hemispherical, conical, truncated conical, pyramidal, truncated pyramidal, cylindrical, prismatic or polyhedral shape.

3. The microfluidic device according to claim 1, wherein a cross section of the protrusion is circular, polygonal, clover-shaped, or an indented closed plane.

4. The microfluidic device according to claim 2, wherein an aspect ratio of the protrusion is from 2:1 to 4:1.

5. The microfluidic device according to claim 2, wherein a tilt angle of a side wall of the protrusion is 50-80°.

6. The microfluidic device according to claim 5, wherein the tilt angle of the side wall of the protrusion is larger than a critical angle at which total internal reflection occurs at an interface between the protrusion and an aqueous solution, an organic solution or air.

7. The microfluidic device according to claim 2, wherein a tilt angle of a side wall of the protrusion is larger than a critical angle at which total internal reflection occurs at an interface between the protrusion and an aqueous solution, an organic solution or air.

8. The microfluidic device according to claim 2, wherein a height of the protrusion is 2-190 $\mu$m.

9. The microfluidic device according to claim 6, wherein a height of the microchannel is 5-200 $\mu$m.

10. A method for analyzing a sample, comprising: injecting a sample into the microchannel of the microfluidic device according to any one of claims 1 to 9; irradiating light from under the microchannel; and analyzing the sample by detecting light emitted from the sample.

[FIG. 1a]

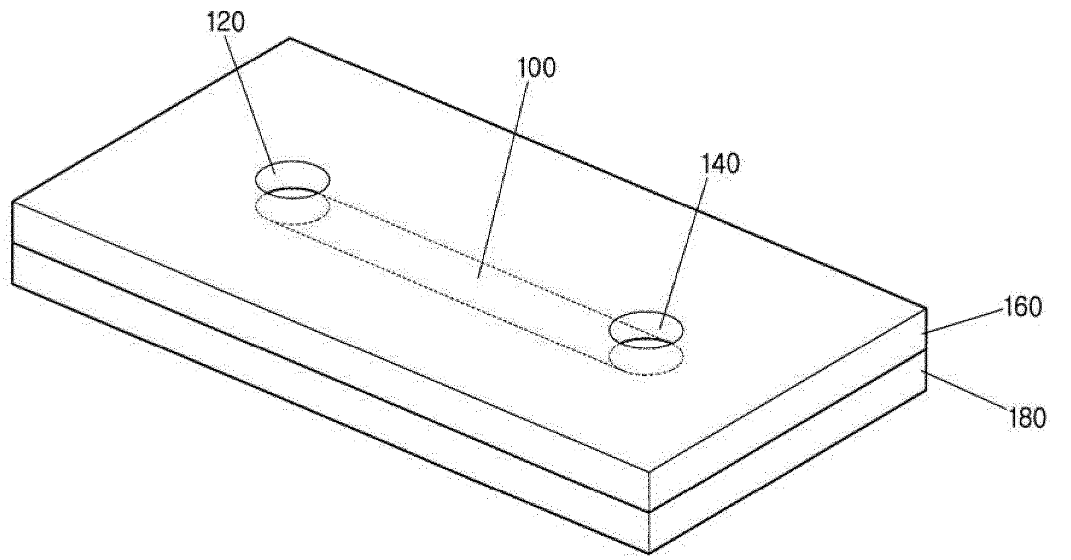


[FIG. 1b]

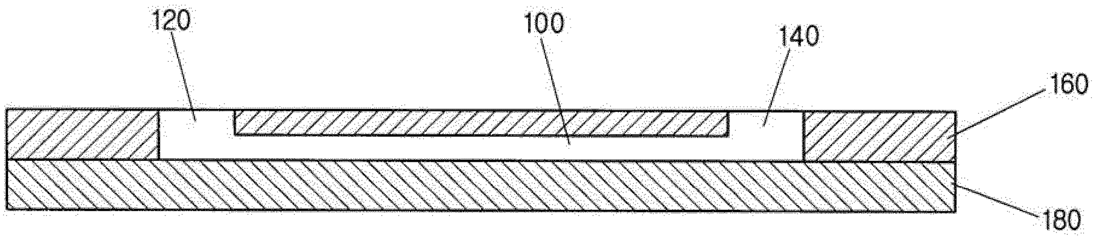


[FIG. 2a]

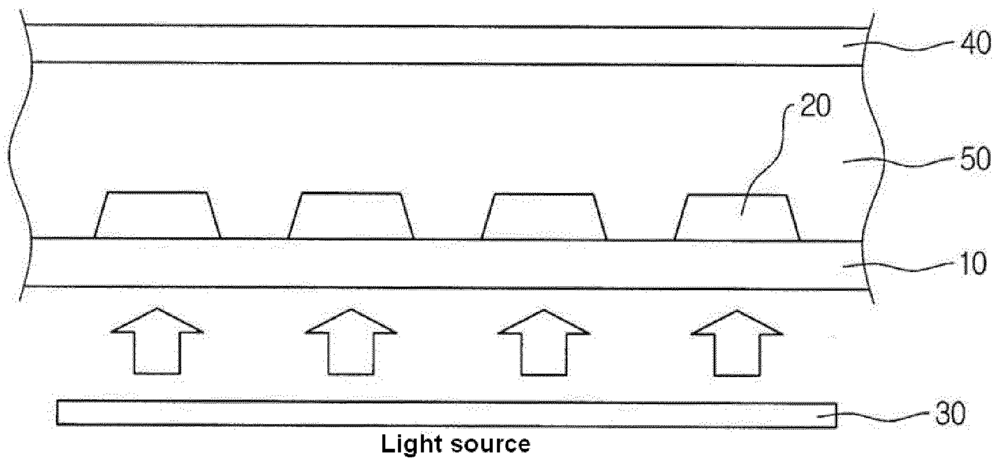

[FIG. 2b]

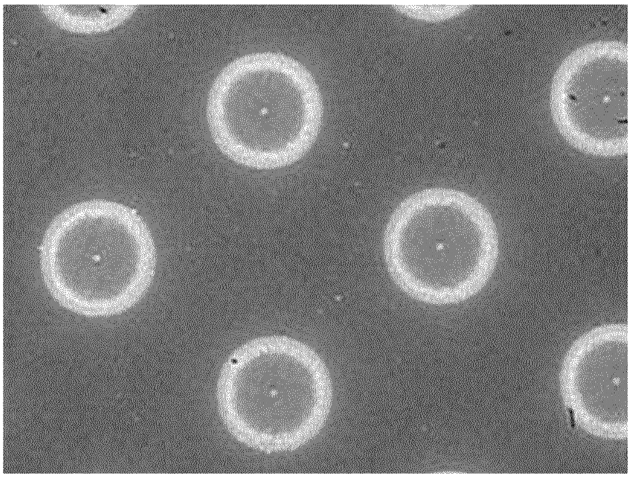

[FIG. 2c]

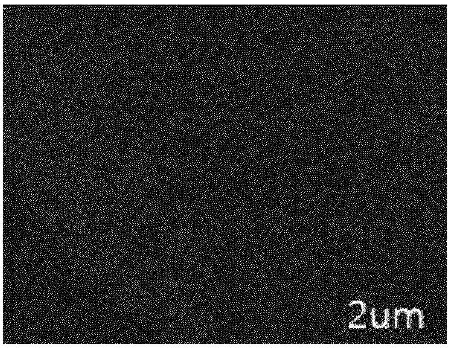


[FIG. 2d]

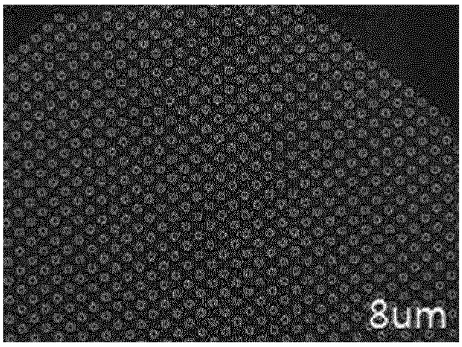

[FIG. 2e]

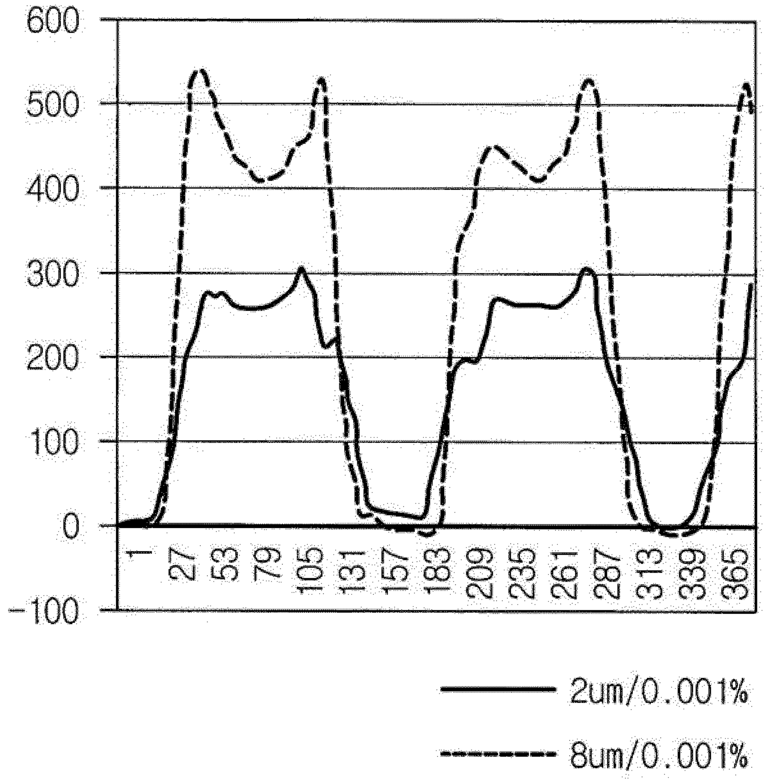
600
500
400
300
200
100
0
-100
1
27
53
79
105
131
157
183
209
235
261
287
313
339
365
2um/0.001%
8um/0.001%

[FIG. 3a]

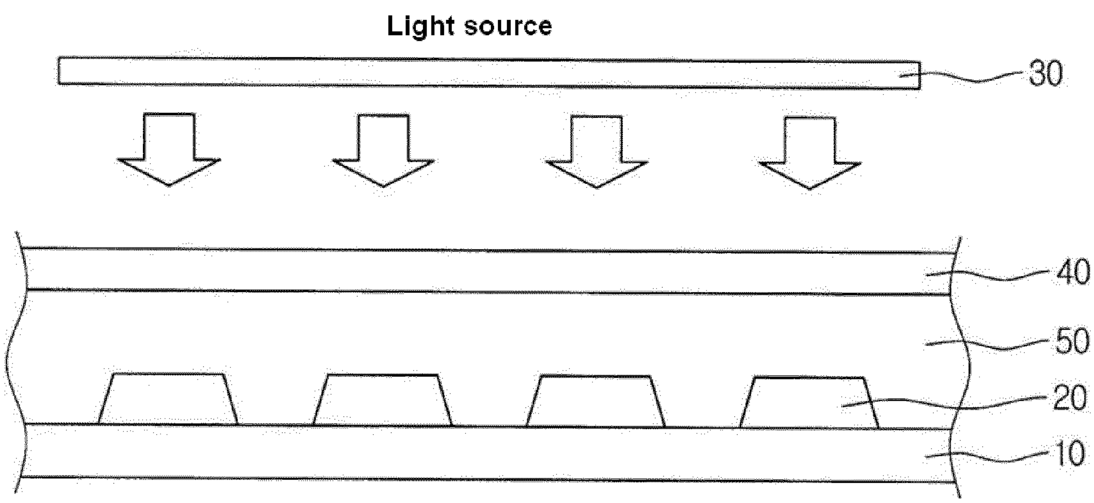
Light source
30
40
50
20
10

[FIG. 3b]

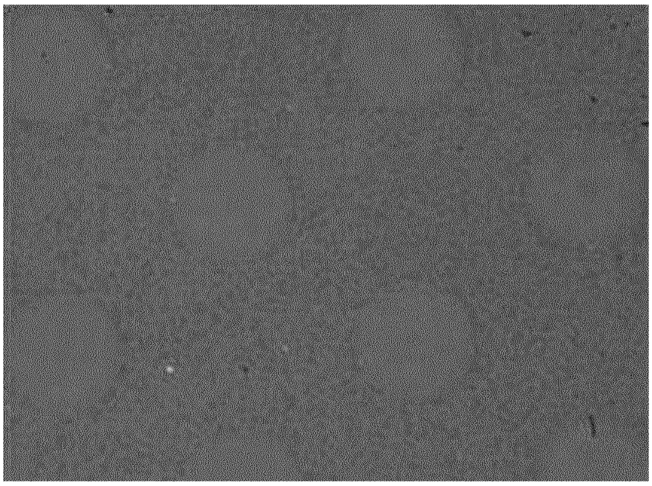

[FIG. 3c]

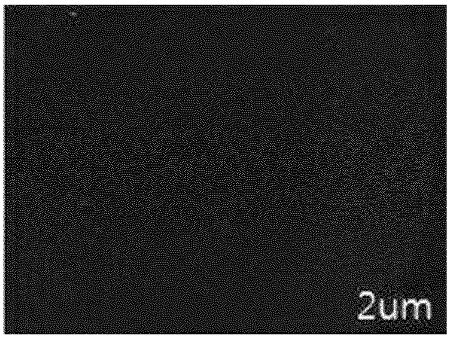


[FIG. 3d]

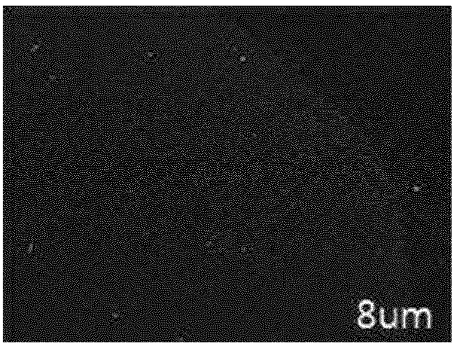

[FIG. 3e]

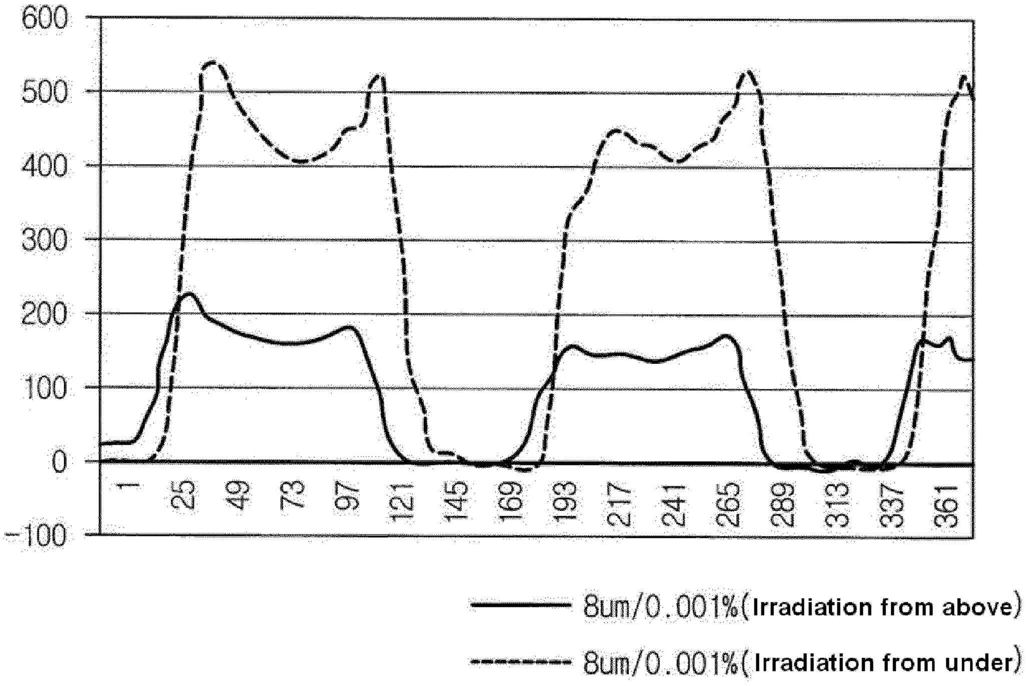
600
500
400
300
200
100
0
-100
1
25
49
73
97
121
145
169
193
217
241
265
289
313
337
361
8um/0.001%(Irradiation from above)
8um/0.001%(Irradiation from under)

[FIG. 3f]

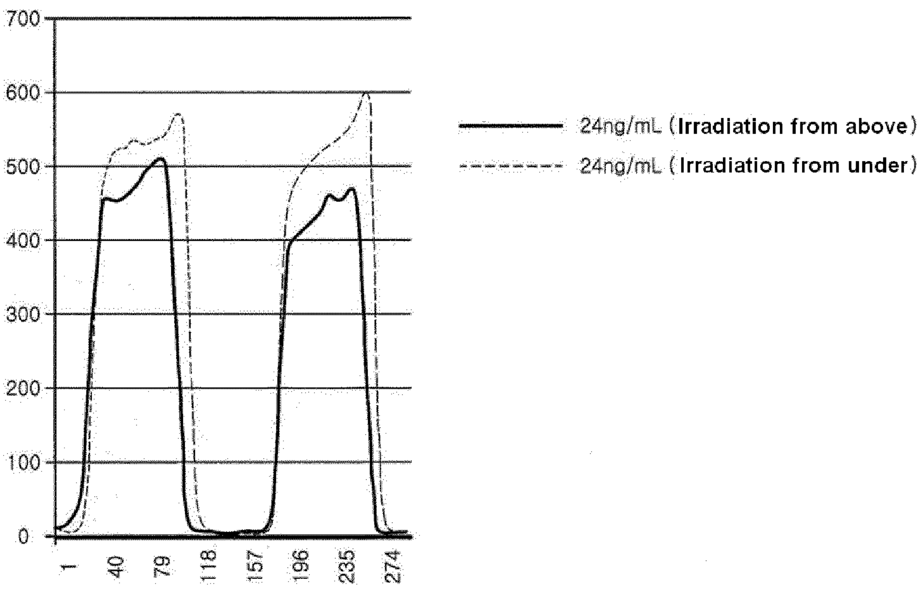
700
600
500
400
300
200
100
0
1
40
79
118
157
196
235
274
24ng/mL (Irradiation from above)
24ng/mL (Irradiation from under)

[FIG. 3g]

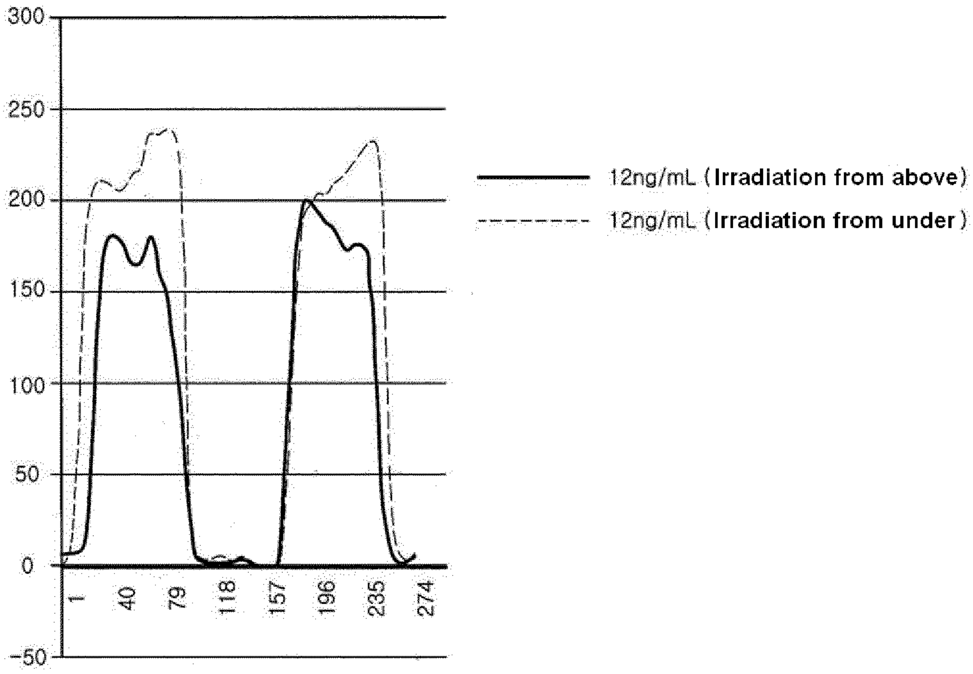
300
250
200
150
100
50
0
-50
1
40
79
118
157
196
235
274
12ng/mL (Irradiation from above)
12ng/mL (Irradiation from under)

[FIG. 3h]

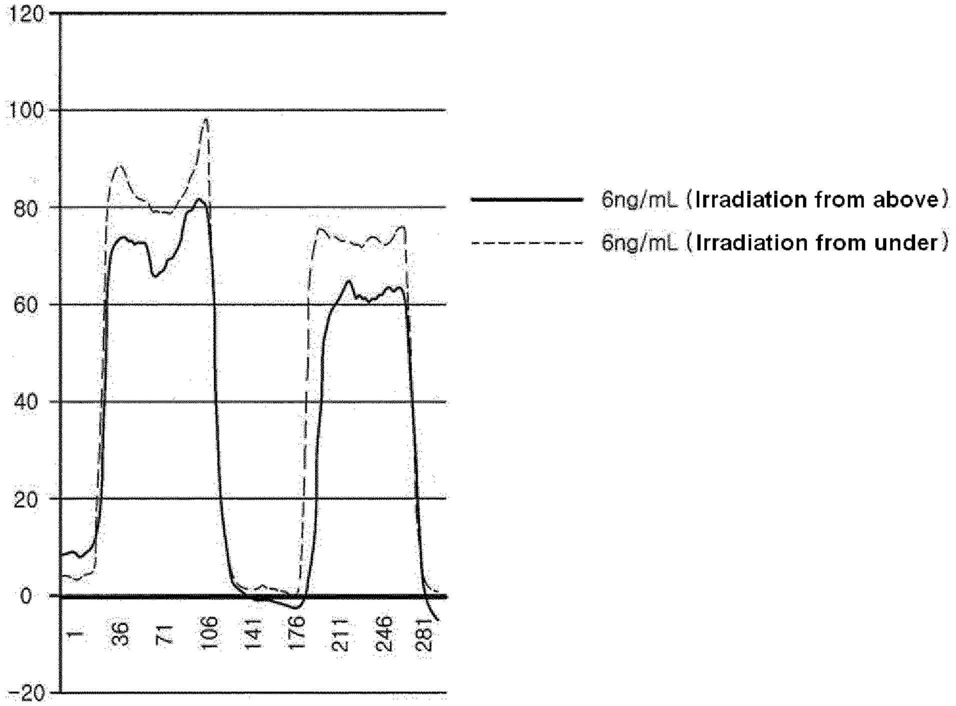
120
100
80
60
40
20
0
-20
1
36
71
106
141
176
211
246
281
6ng/mL (Irradiation from above)
6ng/mL (Irradiation from under)

[FIG. 3i]

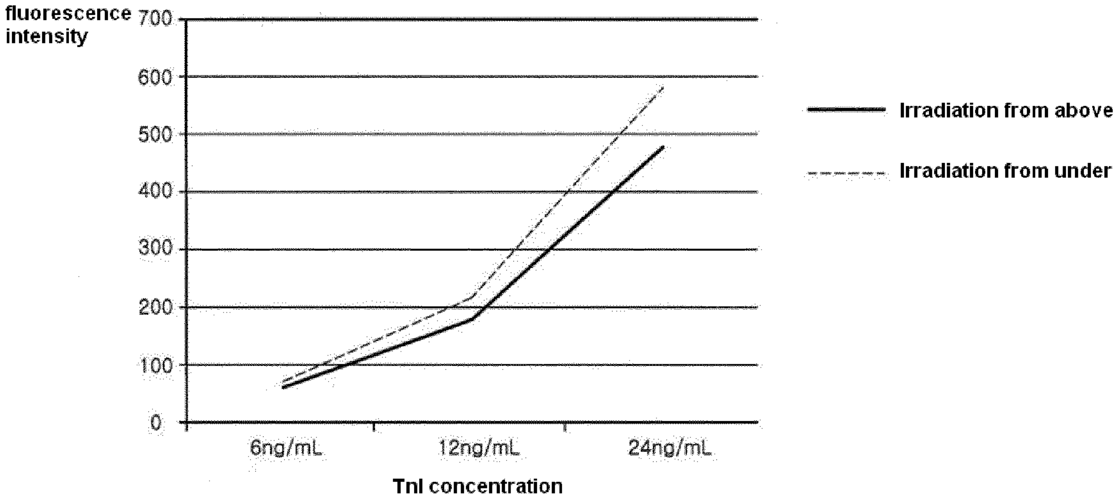
fluorescence intensity
700
600
500
400
300
200
100
0
6ng/mL
12ng/mL
24ng/mL
TnI concentration
Irradiation from above
Irradiation from under

[FIG. 4a]

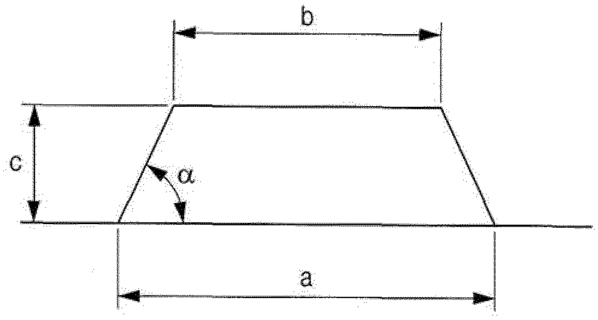


[FIG. 4b]

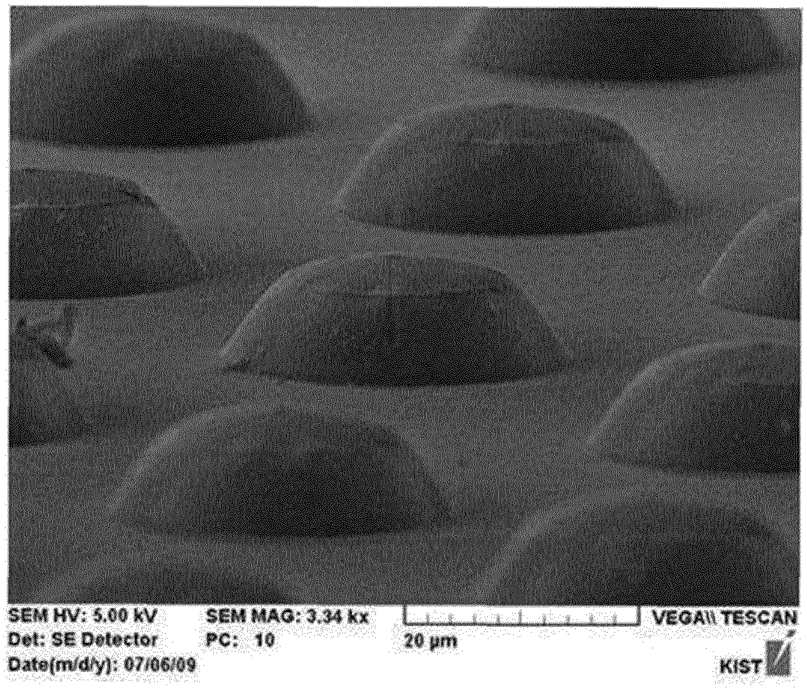


[FIG. 5a]

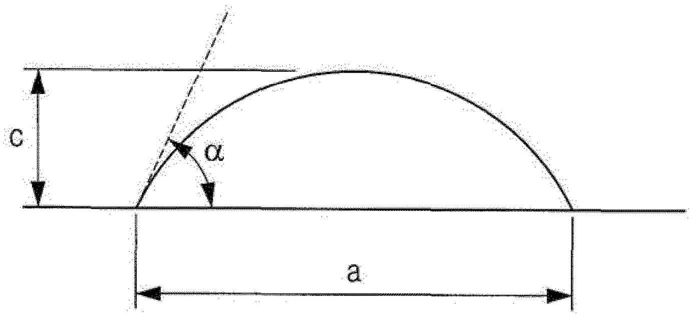

[FIG. 5b]

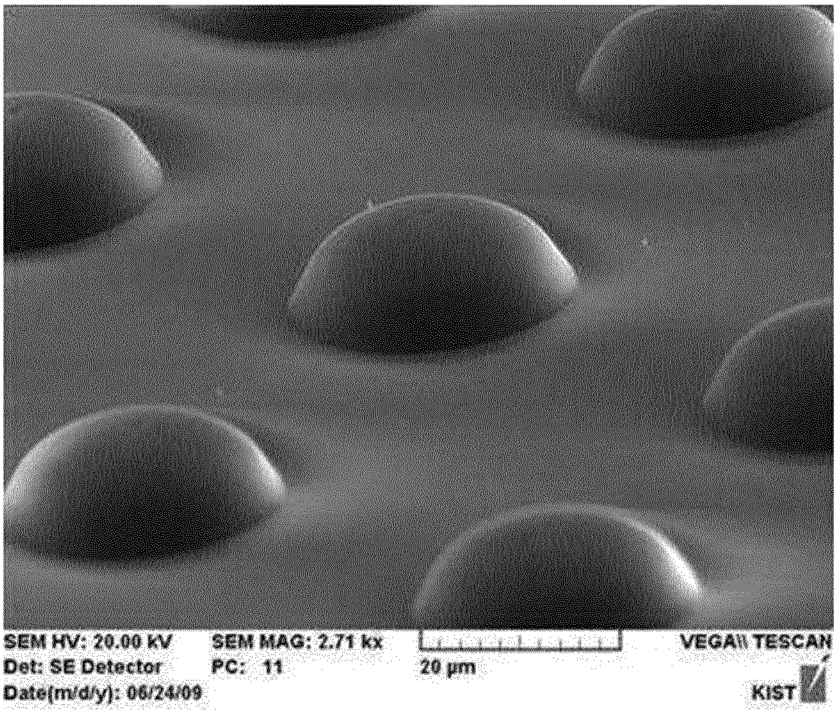
SEM HV: 20.00 kV
SEM MAG: 2.71 kx
20 µm
VEGA\\ TESCAN
Det: SE Detector
PC: 11
Date(m/d/y): 06/24/09
KIST

[FIG. 6]

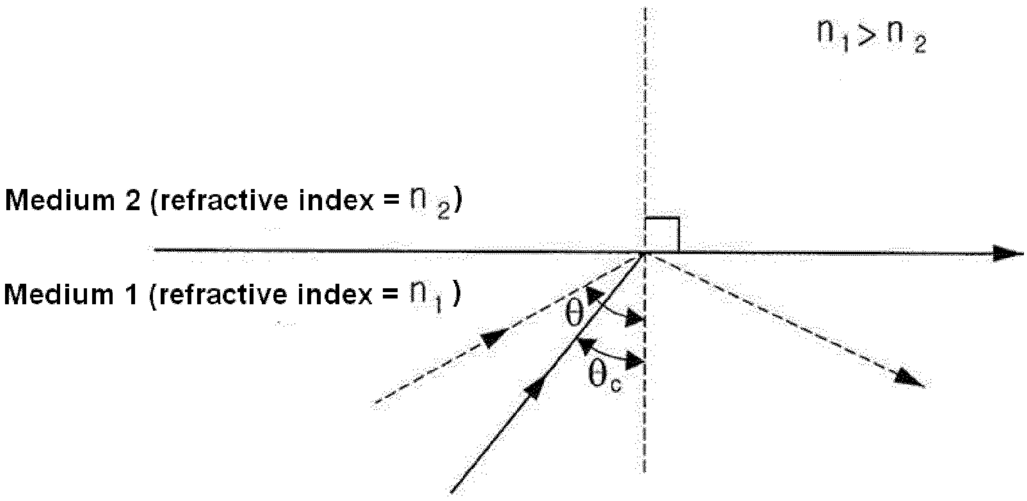
$n_1 > n_2$
Medium 2 (refractive index = $n_2$)
Medium 1 (refractive index = $n_1$)
θ
$θ_c$

[FIG. 7]

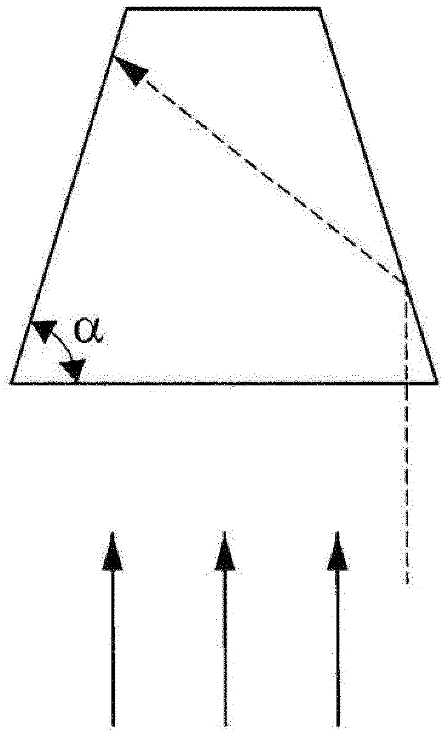


[FIG. 8a]

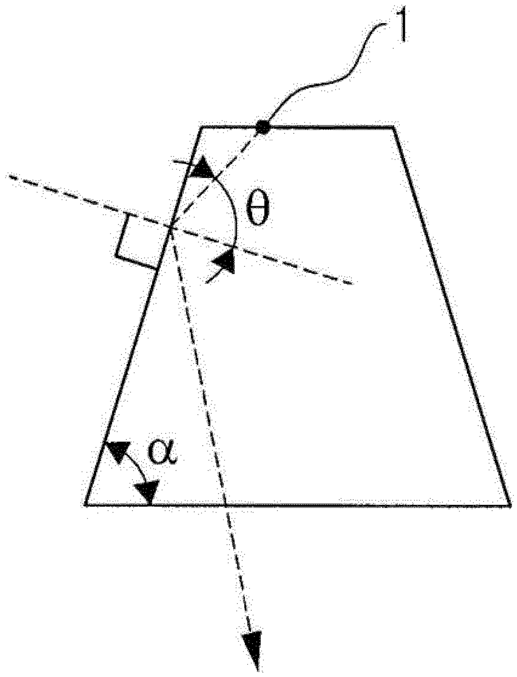

[FIG. 8b]

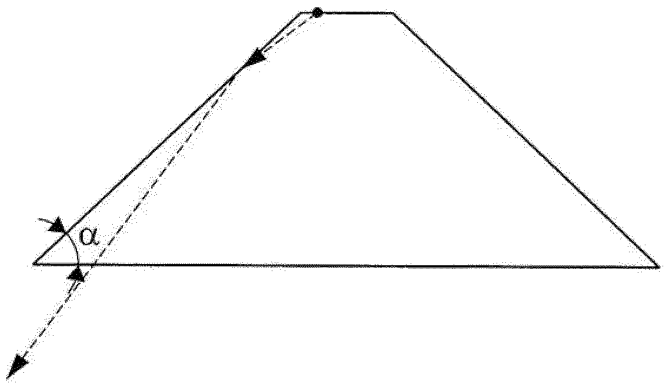


[FIG. 9a]

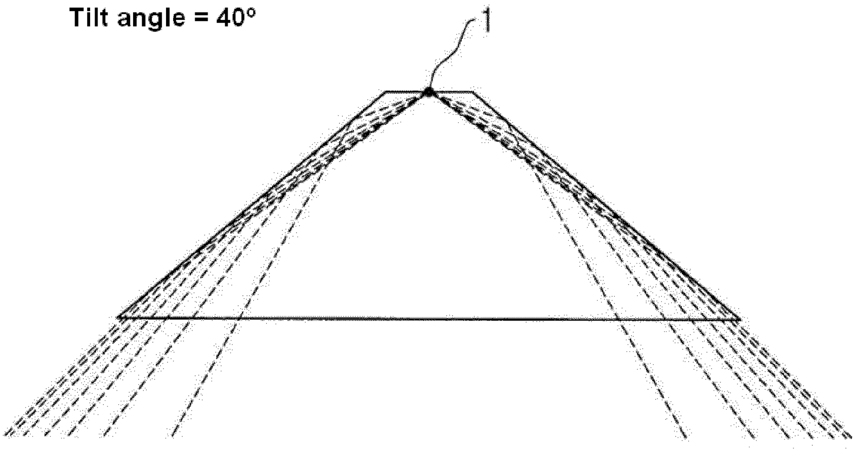


[FIG. 9b]

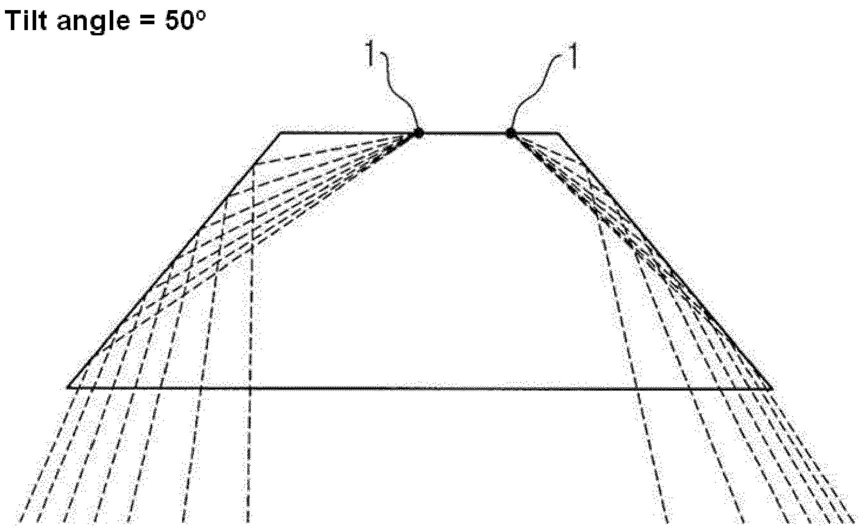

[FIG. 9c]

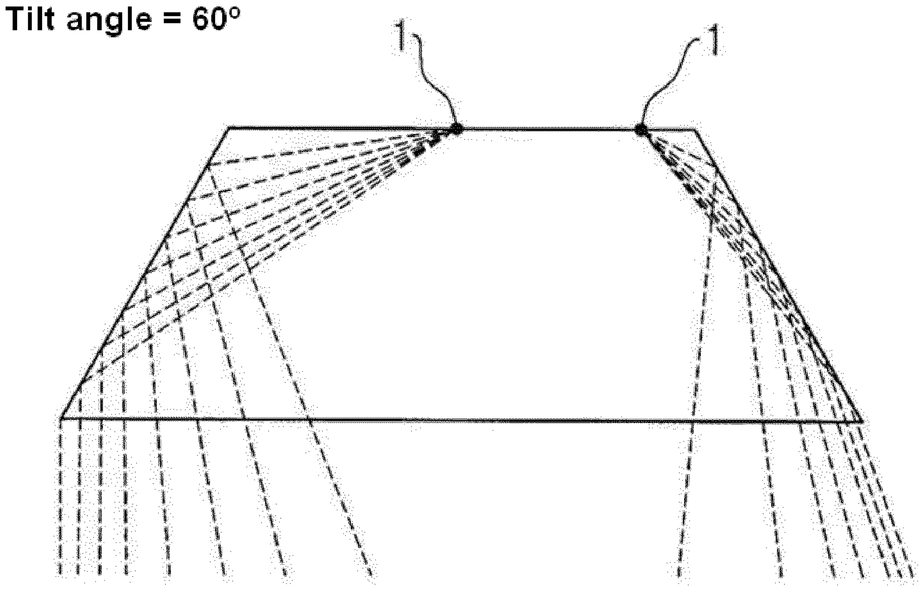


[FIG. 9d]

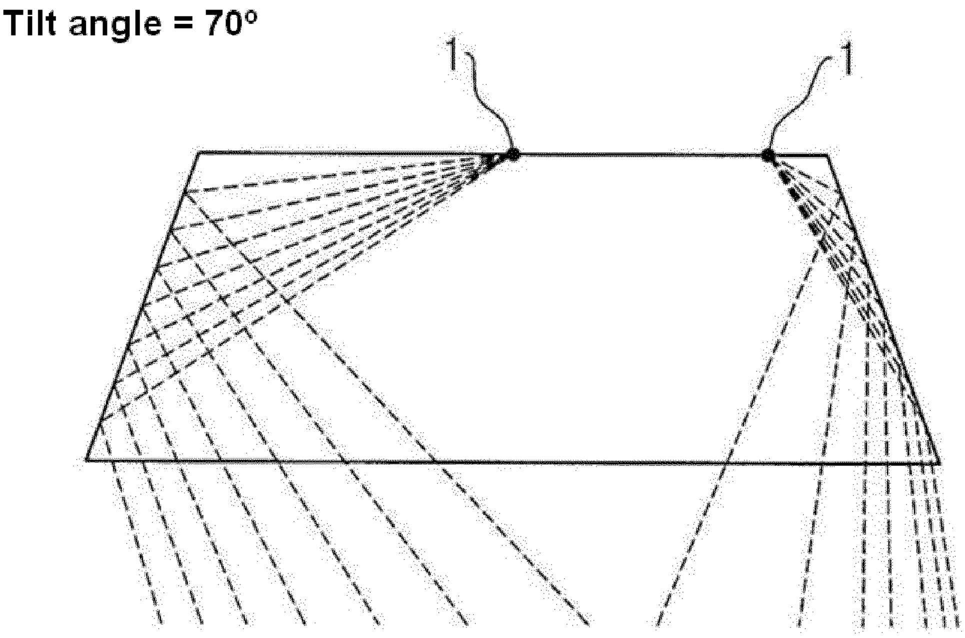

[FIG. 9e]

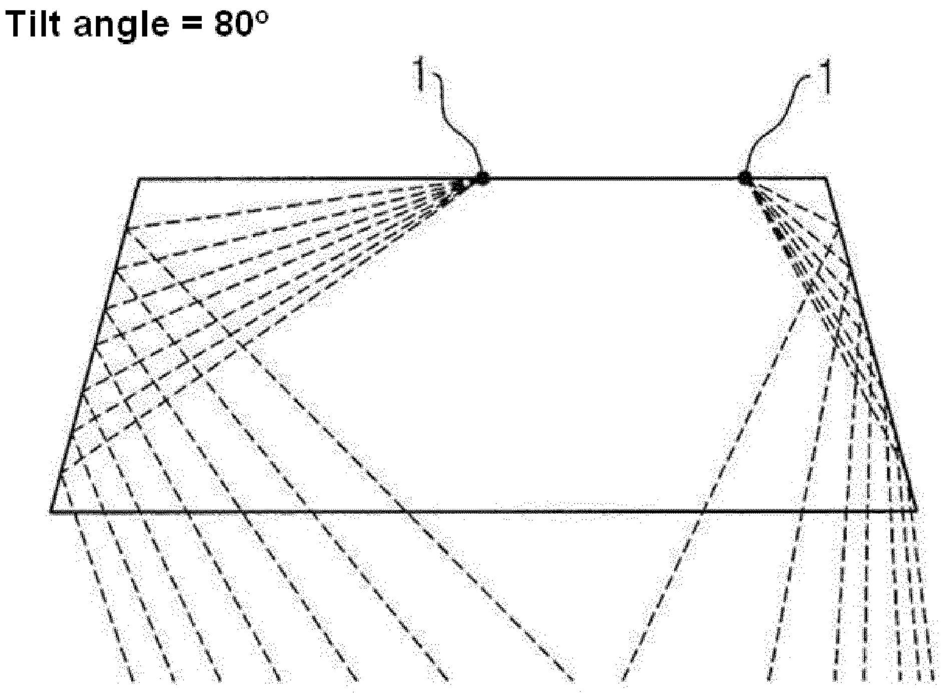

[FIG. 10]

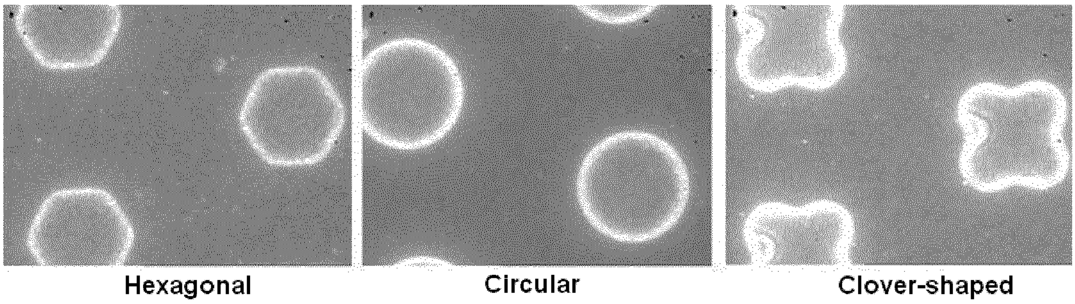


[FIG. 11]